Europäisches Patentamt

European Patent Office

Office européen des brevets

(19)

(11) Publication number: **0 535 239 A1**

## EUROPEAN PATENT APPLICATION
## published in accordance with Art. 158(3) EPC

(12)

(21) Application number: 92906928.4

(22) Date of filing: **18.03.92**

(86) International application number: **PCT/JP92/00328**

(87) International publication number: **WO 92/16846 (01.10.92 92/25)**

(51) Int. Cl.5: **G01N 33/577**, G01N 33/53, G01N 33/531

(30) Priority: **18.03.91 JP 80892/91**
**24.08.91 JP 237134/91**

(43) Date of publication of application:
**07.04.93 Bulletin 93/14**

(84) Designated Contracting States:
**DE FR GB IT NL**

(71) Applicant: **SHISEIDO COMPANY LIMITED**
**7-5-5, Ginza**
**Chuo-ku Tokyo 104-10(JP)**
Applicant: **NIPPONSHOJI CO., LTD.**
**2-9, Kokumachi 2-chome**
**Chuo-ku, Osaka-shi, Osaka 540(JP)**

(72) Inventor: **AMANO, Satoshi, Shiseido**
**Laboratory**
**1050, Nippa-cho, Kohoku-ku**
**Yokohama-shi, Kanagawa 223(JP)**
Inventor: **MASUDA, Yoshiko, Shiseido**
**Laboratory**
**1050, Nippa-cho, Kohoku-ku**
**Yokohama-shi, Kanagawa 223(JP)**

Inventor: **YOSHIDA, Tsuyoshi, Shiseido**
**Laboratory**
**1050, Nippa-cho, Kohoku-ku**
**Yokohama-shi, Kanagawa 223(JP)**
Inventor: **ASAMATSU, Chinatsu, Shiseido**
**Laboratory**
**1050, Nippa-cho, Kohoku-ku**
**Yokohama-shi, Kanagawa 223(JP)**
Inventor: **ITOH, Shigeki**
**2-22-202, Wakayamadai 2-chome,**
**Shimamoto-cho**
**Mishima-gun, Osaka 618(JP)**
Inventor: **FUJIO, Mieko**
**10-5-207, Shintyujyo-cho**
**Ibaraki-shi, Osaka 567(JP)**

(74) Representative: **Patentanwälte Dipl.-Ing. A.**
**Grünecker, Dr.-Ing. H. Kinkeldey, Dr.-Ing. W.**
**Stockmair,**
**Dr. rer. nat. K. Schumann, Dipl.-Ing. P. Jakob,**
**Dr. rer. nat. G. Bezold Maximilianstrasse 58**
**W-8000 München 22 (DE)**

(54) **COLLAGEN ASSAYING METHOD AND KIT.**

(57) A method of assaying collagen with a remarkably enhanced sensitivity by treating a specimen with a chaotropic ion/heparin chelating agent, gelatin and albumin, and a kit therefor.

Effect of NaSCN

Fig. 5

2

[ Technical Field ]

The present invention relates to a method of measuring collagen and measuring tool kit and more particularly, to a method of measuring collagen making use of an immunoreaction.

[ Background Art ]

In measurement of various types of collagen existing in serum, measured values obtained from various types of patients are significantly higher as compared to those obtained from healthy people, and it is known that this comparison is effective for detecting diseases, so that the significance of collagen measurement is now much evaluated.

There are some reports that a radio immunoassay was carried out by using polyclonal antibodies of a human type III collagen N terminal peptides, a human type IV collagen N terminal peptide, 7S and a human type IV collagen C terminal peptide, NCI (Rohde et al., (1979) Eur.J.Clin.Invest., 9.451-459; Hogemann et al., (1984) Clin,Chim.Acta, 144,1-10; and Schuppan et al., (1986) J.Clin.Invest. 78.244 - 248).

Now the possibility of histochemical analysis of diseases using monoclonal antibodies for various types of pathological issues is under investigation, and also for collagen in human blood, there are some reports that enzyme immunoassay was carried out by using monoclonal antibodies (Obata et al., (1989) Clin. Chim. Acta, 181,293 - 303, Japanese Patent Laid Open Publication No.78067/1988, Japanese Patent Laid Open Publication No.246396/1988, Japanese Patent Laid Open Publication No.1553/1990).

Thus, measurement of various types of collagen existing in blood serum has been introduced for diagnosis of diseases.

However, it has turned out that, when measurement of serum collagen concentration is carried out by using such monoclonal antibody as described in Japanese Patent Laid Open Publication No.78067/1988 or Japanese Patent Laid Open Publication No.246396/1988, or when measurement of serum collagen is carried out by using an ordinary enzyme immunoassay method as described in Japanese Patent Laid Open Publication No.1553/1990, reproducibility of the measured values is rather poor, and that, if a human serum is used as a sample to be assessed, the recovery ratio when a collagen is added experimentally is not high.

[ Disclosure of the Invention ]

Accordingly, it is an object of the present invention to eliminate the above-described probrems in the related art and to provide a highly sensitive collagen measuring method with a high recovery ratio as well as a high reproducibility.

To achieve the object as described above, inventors of the present invention made strenuous efforts, found out that the reactivity between collagen included in a sample to be assessed and monoclonal antibodies becomes higher by adding chaotropic ion, and have completed the present invention.

Namely, the method of measuring collagen according to claim 1 in this application is characterized in that a sample to be assessed is treated with chaotropic ions in an immunoassay using a monoclonal antibody against collagen.

The method according to claim 2 is characterized in that chaotropic ions are added so that the concentration is 0.01 M or more.

The method according to claim 3 is characterized in that a sample to be assessed is treated with heparins.

The method according to claim 4 is characterized in that a sample to be assessed is treated with a chelating agent.

The method according to claim 5 is characterized in that a sample to be assessed is treated with gelatins.

The method according to claim 6 is characterized in that a sample to be assessed is treated with albumins.

The method according to claim 7 is characterized in that chaotropic ions are added so that the concentration will be in a range from 0.1 to 1.6 M during an immunoreaction of the serum sample to be assessed.

The method according to claim 8 is characterized in that chaotropic ions are added so that the concentration will be in a range from 0.2 to 0.6 M when a sample to be assessed is pretreated.

The method according to claim 9 is characterized in that heparins are added so that the concentration will be in a range from 250 to 15000 U/ml and/or in a range from 5 to 60 mg/ml during an immunoreaction of a sample to be assessed.

The method according to claim 10 is characterized in that heparins are added so that the concentration will be in a range from 250 to 5000 U/mil when a sample to be assessed is pretreated.

The method according to claim 11 is characterized in that a sample to be assessed is treated with a chelating agent including bivalent cations at the concentration of 1 to 10 mM.

The method according to claim 12 is characterized in that a sample to be assessed is treated with gelatins in an immunoassay using a monoclonal antibody against collagen.

The method according to claim 13 is characterized in that a sample to be assessed is treated with heparins together with gelatins in the immunoassay as described in claim 12.

The collagen measuring tool kit according to claim 14 is characterized in that said measuring tool kit includes;

an antibody coated solid phase in which an antibody against collagen has been coated,

a sample treating agent which includes an enzyme-labelled monoclonal antibody against collagen and chaotropic ions, and reacts to a sample to be assessed and to the aforesaid antibody coated solid phase, and

an enzyme-substrate solution which includes a substrate of an enzyme in the aforesaid enzyme-labelled monoclonal antibody.

The collagen measuring tool kit according to claim 15 is characterized in that said collagen measuring tool kit includes;

a reaction phase wherein an enzyme-labelled monoclonal antibody against collagen, chaotropic ion and a carrier on which a monoclonal antibody against collagen has been coated coexists, and

an enzyme-substrate solution including a substrate of an enzyme in the aforesaid enzyme-labelled monoclonal antibody.

Details of the present invention will be explained hereinunder.

The chaotropic ion used in the assessing method according to the present invention is a univalent anion having a protein denaturing effect represented by such ions as thiocyanate ion, iodine ion, perchlorate ion, bromine ion, nitrate ion, chlorine ion, and salicylate ion; a group of substances having a large ionic radius. Herein, the treatment with chaotropic ions means to add a chaotropic salt such as sodium thiocyanate, sodium perchlorate, potassium iodide, or potassium bromide during an immunoreaction between a sample to be assessed and an antibody or when a sample to be assessed is pretreated, and this operation ensures an excellent result.

It should be noted that chaotropic ions should preferably be added so that the concentration is 0.01 M or more. If the concentration is less than 0.01 M, the effect to raise the sensitivity in assessment is low.

Also, if a sample to be assessed is serum and chaotropic ions are added during an immunoreaction, the chaotropic ions should preferably be added so that the concentration is in a range from 0.1 to 1.6 M. If a sample to be assessed is serum, obstruction to the assessment by a coexisting substance is remarkable, but the sensitivity to collagen can largely be improved by raising the concentration of chaotropic ions to a relatively higher level.

When a serum sample to be assessed is pretreated with chaotropic ions, it is preferable to add chaotropic ions so that the concentration will be in a range from 0.2 to 0.6 M. When the concentration is less than 0.2 M or more than 0.6 M, the effect to raise the sensitivity in measurement is low.

The heparins used according to the present invention is proteoglycan having a sulphonyl such as heparin sodium, chondroitin sulfate A or C sodium salt. Herein, for treatment with heparins, the heparins have only to be added under the existence of chaotropic ions during an immunoreaction or when a sample to be assessed is pretreated, and with this operation, a higher effect can be obtained.

It should be noted that, when heparins are added during an immunoreaction, sometimes an appropriate effect can not be obtained if the concentration of heparins in a sample to be assessed is less than 250 U/ml(or less than 5 mg/ml in case of chondroitin sulfate A or C sodium salt), and also that, if the concentration exceeds 15000 U/ml (or 60 mg/ml in case of chondroitin sulfurate A or C sodium salt), the effect of adding heparins can not be improved any more.

Also, when heparins are added for pretreating a sample to be assessed, if the concentration of heparins in the sample to be assessed is less than 250 U/ml, sometimes an appropriate effect can not be obtained, and also that, if the concentration exceeds 5000 U/ml, often the effect of heparins can not be improved.

Also, the chelating agent including bivalent cations in an assessing method according to the present invention includes a group of substances having the effect to chelate bivalent cations represented by various types of ethylenediaminetetraacetic acid (such as EDTA) and ethylene glycol-0,0'-bis (2-aminoethyl)-N,N,N',N'-tetraacetate (EGTA). Herein, it is preferable to carry out the treatment with a chelate agent for bivalent cations before a sample to be assessed is frozen, but also the effect can be obtained even if the treatment is carried out during a reaction between a sample to be assessed and an antibody.

It should be noted that, if the concentration of a chelating agent is less than 1 mM, sometimes an adequate effect may not be obtained, and also that, if the concentration exceeds 10 mM, improvement of the effect of adding the chelating agent can not be observed.

Also it should be noted that the gelatins used in the assessing method according to the present invention include not only high molecular gelatins, but also hydrolysates having a low molecular weight ( e.g., PA10, 100, 200, and PE 20, 50: all available from Nippi).

In this treatment, if gelatin is added so that the concentration is in a range from 0.2 to 15 % during an immunoreaction, or in a range from 1.5 to 4.5 % when pretreated with a sample to be assessed, a better effect can be obtained. If a concentration of gelatins in a sample to be assessed is less than 1.5 weight %, sometimes an adequate effect can not be obtained, and also if the concentration is more than 4.5 weight %, inprovement of the effect of adding gelatins can not be obtained in many cases.

The effect of adding gelatins is improved by coexistence of heparins.

In the treatment with gelatins and/or heparins according to the present invention, it is preferable to dilute or pretreat a sample to be assessed with a buffer solution containing gelatins and/or heparins.

The albumins used according to the present invention are albumins from animals such as bovine serum albumin or human serum albumin. In this process, it is possible for the albumins to coexist with the aforesaid gelatins by adjusting the concentration, but it is more preferable to add albumins in place of gelatins, and a good result can be obtained by adding albumins so that the concentration is in a range from 0.2 to 15 % during an immunoreaction.

As a monoclonal antibody used in the assessing method according to the present invention, either IgG, F(ab')$_2$ or Fab' may be used as it is.

Also, the collagen, which is an object for measurement according to the present invention, should preferably be human type IV collagen. The human type IV collagen is human basement membrane-related collagen, and includes a collagen complex from endothelial basement membrane.

Also an measuring method, to which the present invention is applicable, includes enzyme immunoassay (EIA), radio immunoassay (RIA) and fluorescence immunoassay (FIA), and the present invention is applicable to a method in which the immunoreaction is carried out in one step or two step.

The function provided by the present invention is not always clear, and although the sensitivity of purified collagen against various types of monoclonal antibodies is high, the present invention provides an extremely high effectiveness when the reactivity with a sample obtained from organism to be assessed is low.

The cases where the reactivity between collagen and an antibody drops include (1) a case where a binding site of the collagen with the antibody is masked, and (2) a case where the reactivity of collagen with an antibody has been suppressed.

The function provided by chaotropic ions relates to the former, namely change of the composition of collagen, while that provided by a bivalent cation chelating agent relates to the latter, namely elimination of the suppressing effect, both of which seem to improve reactivity of collagen with an antibody.

Also, addition of chaotropic ions gives a merit that the reaction temperature during an immunoreaction or when a sample to be assessed is pretreated can be dropped to a room temperature.

[ Brief Description of the Drawings ]

FIG.1 is an explanatory view of a concept of collagen measuring method used in one embodiment of the present invention (3-step sandwich method);

FIG.2 is a calibration curbe of collagen according to the method illustrated in FIG.1;

FIG.3 is an explanatory view of a concept of collagen measuring method in another embodiment of the present invention (1-step sandwich method);

FIG.4 is a calibration curve of collagen according to the method illustrated in FIG.3;

FIG.5 is an explanatory view of effects of chaotropic ions in measurement of purified collagen;

FIG.6 is an explanatory view of effects of chaotropic ions in cases of the 1-step sandwich method and the 3-step sandwich method in measurement of collagen in serum;

FIG.7 is an explanatory view of a relation between the concentration of added heparin sodium and the effect of its addition;

FIG.8 is an explanatory view of a relation between the concentration of added chondroitin sulfate A sodium salt and the effect of its addition;

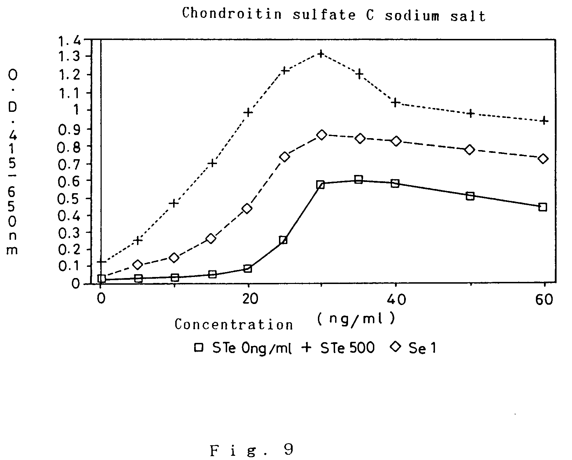

FIG.9 is an explanatory view of a relation between the concentration of added chondroitin sulfate C sodium salt and the effect of its addition;

FIG.10 is an explanatory view of serum's activity for suppressing the sandwich ELISA reaction;

FIG.11 is an explanatory view of a mechanism to prevent appearance of the serum's activity for suppressing the sandwich ELISA reaction when EDTA is added immediately after separation of serum;

FIG.12 is an explanatory view of a mechanism to prevent appearance of serum's activity for suppressing the sandwich ELISA reaction when EDTA is added before serum is frozen and measurement is carried out after the serum is stored for a certain period in the frozen state;

FIG.13 is an explanatory view of a mechanism to prevent appearance of serum's activity for suppressing the sandwich ELISA reaction in plasma to which EDTA was added immediately after the blood was taken out;

FIG.14 is an explanatory view of a mechanism to prevent serum's activity for suppressing the sandwich ELISA reaction when EDTA is added in a blocking buffer solution;

FIG.15 is an explanatory view of a relation between the concentration of gelatins and the effect of its addition in the 1-step sandwich method;

FIG.16 is an explanatory view of a relation between the concentration of added gelatins and the effect of its addition in the 3-step sandwich method;

FIG.17 is an explanatory view of a relation between the concentration of hydrolysates of gelatins and the effect of addition thereof in the 3-step sandwich method;

FIG.18 and FIG.19 are explanatory views of relations between the densities of added gelatins and heparin sodium and the effect of addition thereof in the 3-step sandwich method when the gelatins and heparin sodium coexist;

FIG.20 is an explanatory view of the effect of adding only heparin sodium in the 3-step sandwich method;

FIG.21 is an explanatory view of a relation between the concentration of added albumin and the effect of its addition; and

FIG.22 is an explanatory view of a result of measurement of human type IV collagen in blood of patients having different diseases respectively, when gelatin, heparin sodium, EDTA and NASCN coexist.

[ Best Mode for Carrying out the Invention ]

A preffered embodiment of the present invention will be explained hereinunder with reference to the accompating drawings. It should be noted that the present invention is not limited to the embodiments described below. Unless otherwise specified, a compounding ratio is a weight %.

In the embodiments, EIA. sandwich method was used as an immunoassay.

The 3-step sandwich method and the 1-step sandwich method, which should preferably be used according to the present invention, will be explained hereinunder, prior to description of the embodiments.

3-step sandwich method

The 3-step sandwich method is carried out as described below.

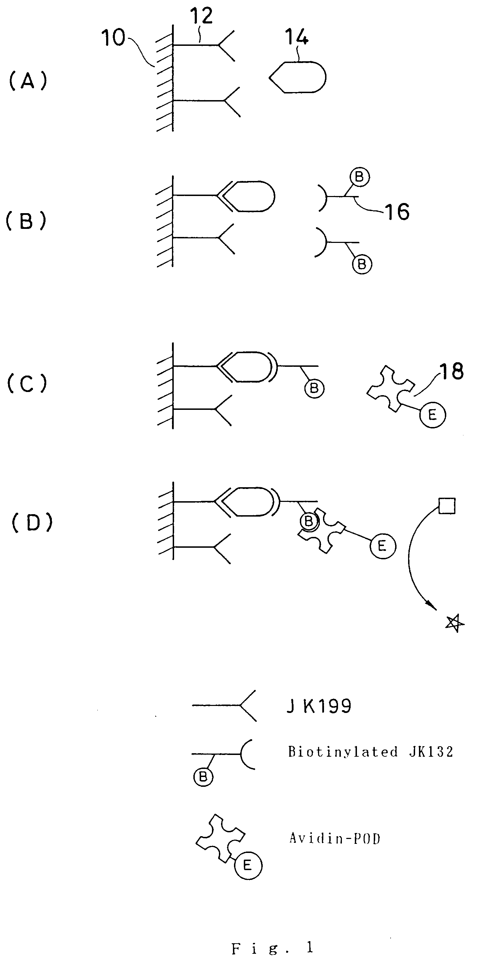

Namely, as shown in FIG.1 (A), an antibody 12 is combined to a well wall 10, and collagen 14 in a sample to be assessed is combined to this coated antibody 12.

Then, a biotinylated antibody 16 is combined to the collagen 14 (FIG.1 (B)).

And, avidin-POD 18 is combined to the biotinylated antibody 16 (FIG. 1 (C)) to promote the chemical reaction with an enzyme active site of the avidin-POD 18, and a quantity of collagen is measured by checking the chemical reaction rate (FIG.1 (D)).

In this 3-step sandwich method, the following processes are required;
1) Preparation of an antigen
2) Preparation of two types of monoclonal antibodies against the aforesaid antigen
3) An enzyme laveling for one of the monoclonal antibodies described above
4) Corting of the another monoclonal antibody described above to a solid phase
5) Actual measurement

1) Preparation of an antigen

As an antigen used as an object for measurement according to the present invention, preparation of pepsinized human type IV collagen was carried out as described below.

2750 g of fresh human placenta was cut off, washed, and homogenized by polytron homogenizer, and then was treated in a refrigerated centrifuge (10,000 × g, for 40 minutes). Precipitate was collected and suspended in 0.02 M sodium phosphate solution, and then refrigerated centrifugation was repeated 3 times.

Obtained precipitate was suspended in 0.5 M acetic acid 41 with the pH adjusted to pH 3 with HCl, pepsin was added to the solution and the solution was left for 7 days at 4°C. The supernatant was collected by centrifugation (10,000 × g, for 1 hour), and then Tris and sodium hydroxide were added to adjust the pH to 8.5 to inactivate pepsin, and the solution was left for 2 days. After neutralization, sodium chloride was added so that the concentration would be 4 M, and furthermore glacial acetic acid was added to adjust the pH to 3, and the solution was left overnight. After centrifugation (10,000 × g, for 1 hour), the supernatant was collected, 0.5 M of acetic acid was added to the precipitate, and the solution was stirred for 5 hours. After centrifugation (10,000 ×g, for 1 hour), the precipitate was collected, sodium chloride was added so that the concentration would be 1.2 M and the solution was left overnight. After centrifugation (10,000 × g, for 1 hour), the precipitate was collected, 0.5 M acetic acid was added to dissolve the precipitate, and only the dissolved portion (supernatant) was collected. The supernatant was dialyzed against water, and the precipitate was removed by means of centrifugation. Tris-sodium chloride solution was gradually added to the obtained supernatant so that finally concentration of Tris would be 0.1 M and that of sodium chloride be 1.0 M. The portion not dissolved was removed by means of centrifugation, and the supernatant was salted out against 0.005M tris-1.5 M sodium chloride solution. After centrifugation, the precipitate was collected, and was dissolved in 0.05 M tris-1.5 M sodium chloride solution. The product was purified by a phosphocellulose column chromatography, and 235 mg of human type IV collagen was obtained. Collagen from the basement membrane is included in this human type IV collagen.

2) Preparation of antibody

Preparation of an antibody against the aforesaid antigen was carried out as described below.

(a) Preparation of immunized spleen cells

0.1 ml of phosphate buffer solution containing 100 $\mu$g of human type IV collagen thus obtained was prepared, and the solution was injected into a BALB/C mouse by means of intraperitoneal injection. One week later, the second immunization was carried out according to the same procedure. In the first and second immunization, 0.1 ml of BACTO-Adjuvant (produced by DIFCO) was injected into the mouse simultaneously by means of intraperitoneal injection. Furthermore one week later, additional immunization was carried out by injecting the aforesaid human type IV collagen into the mouse. In 72 hours after the final additional immunization, a spleen was taken out from this mouse under germfree conditions, cut into pieces by scissors, which were passed through a mesh, and thus a suspension of lymphocytes was obtained. In order to remove erythrocyte mixed therein, cells were suspended in a buffer for hemolysis, and then centrifuged for 5 minutes at 1500 rpm, and washed by RPMI-1640 culture medium 2 times. On the other hand, mouse myeloma cells (SP 2/0) as confluent cells was cultivated in a RPMI-1640 culture medium containing 10% fetal bovine cell (FCS) for two days before confluence under the conditions of 10% $CO_2$ and 37°C. A culture medium containing 100 × 10$^6$ of spleen cell and that containing 100 × 10$^6$ of myeloma cell were mixed, the mixture was centrifuged for 30 minutes at 1500 rpm, the supernatant was thrown away, 1 ml of 50% polyethylene glycol 4000 (produced by Merc Inc.) was dripped drop by drop to unbind the cells. One minute later, 1 ml of the RPMI-1640 culture medium containing FCS was slowly dripped and mixed the cells, and the same operation was repeated for 1 minute. Furthermore, 7 ml of RPHI-1640 culture medium containing FCS was slowly added for 3 minutes, rotating the centrifugal tube. At this point of time, additionally the culture medium was added so that 200 × 10$^6$ of cell would be contained in 50 ml of the culture medium, and this cell suspension was poured by 0.1 ml into each well on a 96 well micro plate respectively, and cultured therein under the conditions of 10% $CO_2$ and 37°C (and 100% relative humidity). it should be noted that phagocyte from mouse had been put in each well on the micro plate at the rate of 10$^4$ well, also that HAT (hypoxanthin, aminobuthelin, thymidine) had been added to the RPMI-1640 culture medium containing bovine serum before the medium was poured into each well on the micro plate. With this improved method, it was not necessary to change the culture medium for at least 5 days, a risk of contamination by bacteria was reduced, and colonies of hybridoma were observed by around 5th day after start of the experiment. In 10 to 25 days, 0.2 ml of the supernatant of the culture medium was taken and used for screening the target antibody. Screening was carried out according to a method introduced in "Collagen Experiment Method" (Kodansha, p.496). Namely, a drop of a phosphate buffer solution containing the human type IV collagen at the ratio of 100$\mu$g/ml was put in each well on a micro plate made of polyvinyl chloride respectively, and the buffer solution was left there overnight under 4°C. After the human type IV collagen solution was removed, 3 drops of 10% bovine serum - phosphate buffer solution were added to each well with a pipet respectively, and blocking was carried out for 1 hour. After the blocking solution was removed, one drop of supernatant in each hybridoma colony was added to a specified well respectively. After the reaction for 1 hours, the supernatant in each well was removed and the cells were washed with the phosphate buffer solution 3 times.

As a secondary antibody, FITC-labelled (fluorescent isothiocyanate)-labelled anti-mouse IgG ( $F(ab)_2$ fragment) solution was added and left therein for 30 minutes, and then the secondary antibody solution was removed and the cells were fully washed. Of the supernatant of the above-described samples to be assessed, those emitting fluorescence were selected, 3 types of hybridoma corresponding to the supernatant were obtained, and these hybridomas were cloned according to the limiting dilution method. At first, the samples were diluted with a culture medium so that the number of hybridomas in a well would be 0.3, 1.0 and 3 respectively. In this step, dilution was carried out very accurately by adding 1 million of thymic cell to each well respectively. The micro plate was kept under the conditions of 37 °C, 10% $CO_2$, and 100 % relative humidity. The wells were checked with a magnifying glass on the 5th day, wells having 1 colony therein were checked, the cells were cultured for 2 weeks, and then the limiting dilution was repeated according to the previous procedure. This operation was repeated 3 times, and finally one type of colony was selected, and the colony was removed to ordinal culture dishes and each colony was cultured with 5 ml of a culture medium respectively until the number of cells increased up to $5 \times 10^6$/ml. The culture medium for the colony was removed to a centrifugal tube and centrifuged for 5 minutes at 1500 rpm to separate the sample to supernatant and cells, and 0.5 ml of RPMI-1640 culture solution was slowly added anew to the cells to suspend them. Then, RPMI-1640 culture medium containing 4.5 ml of FCS was added to the suspension of cells, and subculture was carried out. Namely, $1 \times 10^6$ of cells were suspended in an RPMI culture medium containing 5 ml of bovine serum, and the cells were cultured under the conditions of 5% $CO_2$, 37°C and 100% relative humidity. The number of cells increased to $5 \times 10^6$ in 60 hours, and culture of one generation was over with this operation. The cells were separated from the culture medium by a centrifugal separator, and the cells were diluted by adding a new culture medium. Then, 4 ml of culture medium was added to 1 ml of the cell culture medium in which the cells had been diluted to the level of 1 $\times 10^6$/ml. The subculture was carried out as described above. 50 ml of culture medium in which the cells had been cultured by 10 generations was centrifuged, and $5 \times 10^7$ of cells were obtained, and the cells were suspended in a culture medium 51.

The suspension was divided and poured into 10 rotary culture bottles and cultured therein for 5 days under the conditions of 5 % $CO_2$, 37°C, 100 % relative humidity and rotational speed of 1 rpm. The cells were removed from the culture media in which the number of cells had increased to a level of $5 \times 10^6$/ml, the culture media were sucked and filtered, ammonium sulfate was added to the filtrate so that the concentration would be 45 %, and precipitate was obtained. The precipitate was furthermore purified with protein A. 73 mg of monoclonal antibody against the human type IV collagen was obtained as powder from totally 151 culture media. It was found out by means of SDS- polyacrylamide gel electrophoresis that this monoclonal antibody against human type IV collagen made a single band, and the monoclonal antibody was named JK 199.

Furthere, 92mg of other monoclonal antibody against the human type IV collagen was obtained as powder from totally 151 culture media by same method described above.

It was found out by means of SDS-polyacrylamide gel electrophoresis that this monoclonal antibody against human type IV collagen made a single band and the monoclonal antibody was named JK132.

(b) Immunoglobulin subclasses of JK 199 antibody and JK 132 antibody

It was found out by means of ouchterlony double immunodiffusion method that both of the immunoglobulin subclasses of JK 199 antibody and JK 132 antibody were IgG 2a.

(c) Reactive specificities of JK 199 antibody and JK 132 antibody

Various types of antigen were dissolved in phosphate buffer solution so that the concentration of each antigen would be 1μg/ml, and the solutions were dripped accurately by 0.1 ml into each well on a micro plate made of polyvinyl chloride, and the samples were left overnight under 4°C. After the antigen solutions were removed, 0.5 ml of 10 % bovine serum was added to each well, and blocking was carried out for 1 hour. After the blocking solution was removed, 0.1 ml of JK 199 solution with the concentration of antibody adjusted to 10 μg/ml was dripped into each well, and the samples were left for 1 hour so that the reaction would proceed. After the samples were well washed with phosphate buffer solution, 0.2 ml of peroxidase-labelled antibody against mouse IgG ( produced by Cappel Inc.) was added as a secondary antibody to each well respectively, and the mixtures were left for 1 hour. After the secondary antibody solution was removed, the samples were washed with phosphate buffer solution 4 times, and then substrate solution containing 0.001% hydrogen peroxide was added by 0.1 ml to each well. The substrate solution was prepared just before its use by dissolving o-phenylendiamine in 0.1 M citric acid - 0.2 M phosphate buffer solution (pH 4.8) so that the concentration of phenylendiamine would be 0.4 mg/ml. Then in 10 minutes after the operation described above, 20 $\mu$l of 8M sulfuric acid solution was added to each well to stop the reaction, and absorbance at 492 nm was measured with a microplate photometer. The result is shown in Table 1.

Table 1

|  | SP2/0 | JK199 | JK132 |
|---|---|---|---|
| Human type I collagen | 0.01 | 0.07 | 0.03 |
| Human type III collagen | 0.01 | 0.07 | 0.02 |
| Human type V collagen | 0.01 | 0.09 | 0.04 |
| Human type IV collagen | 0.03 | 1.28 | 1.07 |
| Bovine type IV collagen | 0.04 | 0.05 | 0.07 |
| Human fibronectin | 0.01 | 0.02 | 0.03 |
| Mouse laminin | 0.08 | 0.07 | 0.07 |
| Human laminin | 0.10 | 0.09 | 0.10 |
| * The values indicate an absorption degree of each sample assuming that an absorbance at 492nm of the phosphate buffer solution used in the experiment was 0.00. <br> * SP2/0 is a culture medium of myeloma cells as a control sample. | | | |

As shown in Table 1, JK 199 and JK 132 specifically recognized the type IV collagen which is a collagen fraction from a human basement membrane, but did not recognize other types of collagen fraction; namely human type I collagen fraction, human type III collagen fraction, bovine type IV collagen fraction, and also did not recognize human fibronectin, human laminin, nor mouse laminin.

3) Labeling for monoclonal antibody

According to the present invention, biotinylated monoclonal antibody was prepared as a labelled monoclonal antibody.

Namely, the monoclonal antibody fraction obtained according to the monoclonal antibody preparing method (b) described above was added to 0.1 M sodium bicarbonate so that the concentration would be 1 mg/ml. To 1 ml of this solution, a solution prepared by dissolving N-hydroxysucinimidobiotin in dimethylsuloxide so that the concentration would be 1 mg/ml was added by 0.06 ml, and the mixture was left, being stirred, for 4 hours under the room temperature so that the reaction would proceed. Then, the solution was fully dialyzed against phosphate buffered saline (pH: 7.4), and stored in cooled or frozen state.

It should be noted that, by adding avidinated peroxidase obtained from horseradish to this biotinylated monoclonal antibody, the biotinylated monoclonal antibody was enzyme-labelled by the peroxidase.

By adding 2,2'-azino-di[3-ethyl-benzthiazoline] sulfonate (ABTS) as a substrate, activity of peroxidase, namely the biotinylated monoclonal antibody can be assessed.

4) Immobiligation of monoclonal antibody to solid phase

The monoclonal antibody (JK 199) against human type IV collagen obtained according to the monoclonal antibody preparing method (b) described above was dissolved in a phosphate buffered saline (pH: 7.4), and the concentration was adjusted to 14.5 $\mu$g/ml. 100 $\mu$l of this antibody solution was added to each well on a microplate made of polystyrene as a solid phase carrier for coating, and the samples were treated for 24 hours at 37°C, and then a blocking buffer solution prepared by diluting Block Ace (produced by Dainihon Seiyaku) to 1/4 with phosphate buffered saline (pH: 7.4) was added by 350 $\mu$l to each well, the mixtures were treated for 1 hour for blocking at 37°C, well dried, and stored in frozen state. When the microplate is used, temperature is raised to the room temperature before start of the experiment, and then the microplate is used for assay.

5) Actual measurement

A sample diluted by a blocking buffer solution is added by 100 $\mu$l to each well on a microplate to which antibody has been coated respectively. After incubation (immunoreaction) for 2 hours at 37°C, the samples are washed with phosphate buffered sline (pH: 7.4, washing buffer solution) containing 0.05 % Tween 20 (Refer to FIG.1 (A)).

Then, 0.2 $\mu$g/ml of the purified monoclonal antibody (biotinylated JK 132) against the human basement membrane associated collagen obtained according to this quantifying method (a) and 100 $\mu$l of washing buffer solution (biotinylaed antibody solution) containing mouse IgG by 2 $\mu$g/ml are added to one well, and after incubation (immunoreaction) for 1 hour under 37°C, the samples are washed with washing buffer solution (FIG.1 (B)).

Then, washing buffer solution (av-POD solution) containing avidin-POD (produced by Vector Laboratories) by 0.5 $\mu$g/ml is added by 100 $\mu$l to each well, and after incubation (immunoreaction) for 30 minutes at 37°C, the samples are washed with washing buffer solution (FIG. 1 (C)).

Then, a peroxidase substrate system (produced by Kirkegaard & Perry Laboratories) containing 2,2'-azino-di [ 3-ethyl-benzothiazoline] as the substrate is added by 100 $\mu$l to each well, and after incubation (coloring reaction) for 30 minutes at the room temperature, absorbance at 405 nm is observed by a microplate reader, and a difference between absorbance at the samples and that observed in blind test is checked (FIG.1 (D)).

A quantity of type IV collagen corresponding to an absorbance of each sample was obtained from an calibration curve prepared by using a standard sample (See Table 2 for the measurement conditions).

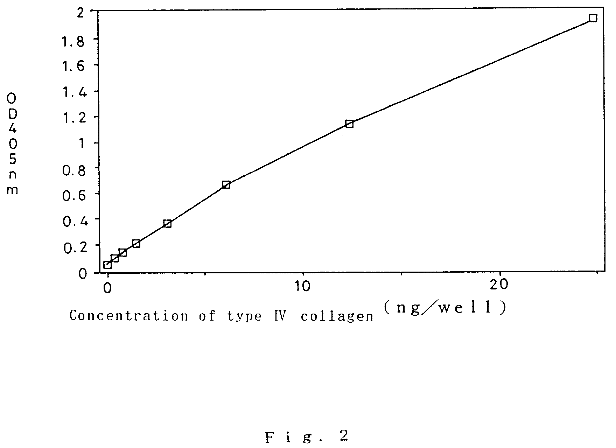

An calibration curve for human type IV collagen is shown in FIG.2. As clearly shown in this figure, the quantitative sensitivity was 0.391 ng/0.1 ml, while a range of determination was from 0.391 to 125 ng/0.1 ml.

Table 2

|  | Samples | Standard solution for calibration curve |
|---|---|---|
| Heat-treated sample Antibody coated microplate | 100 $\mu$l 2 wells | 100 $\mu$l 2 wells |
| Primary immunoreaction | Statically left for 2 hours at 37°C after mixing | |
| Washing | Washed with 350 $\mu$l of washing buffer solution 3 times | |
| Biotinylated antibody solution | 100 | $\mu$l 100 $\mu$l |
| Secondary immunoreaction | Statically left for 2 hours at 37°C after mixing | |
| Washing | Washed with 350 $\mu$l of washing buffer solution 3 times | |
| av-POD solution | 100 $\mu$l | 100 $\mu$l |
| Washing | Washed with 350 $\mu$l of washing buffer solution 3 times | |
| Substrate solution | 100 $\mu$l | 100 $\mu$l |
| Coloring reaction | Statically left for 30 minutes at the room temperature | |
| Colorimetry | Measurement of absorbance at 405 nm | |

1-step sandwich method

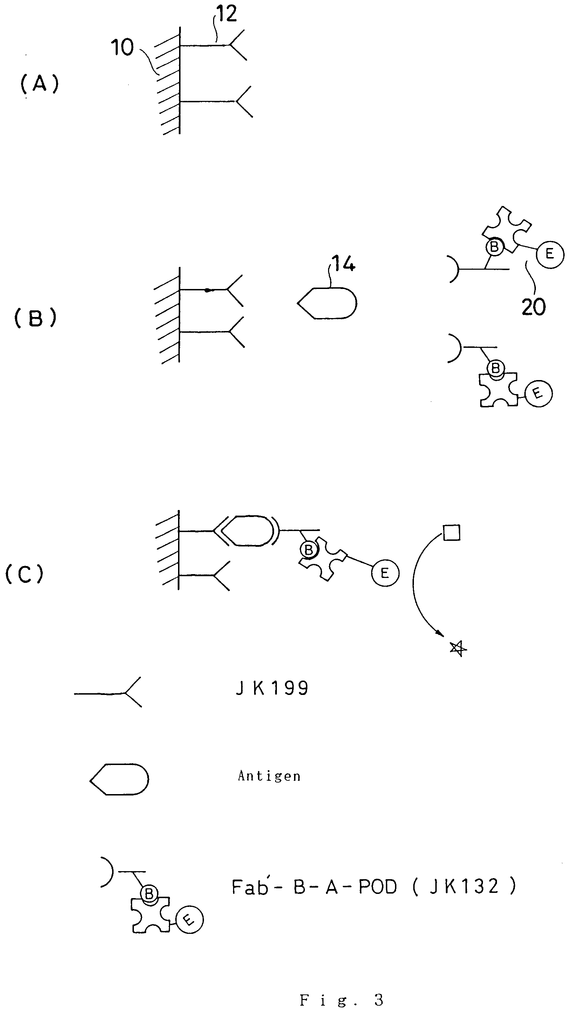

The 1-step sandwich method is carried out as follows. Namely, an antibody 12 is combined to a well wall 10 as shown in FIG.3 (A). Then, collagen 14 in the sample and biotinylated antibody - avidin POD complex(Fab'- B-A-POD) 20 are combined to this antibody (FIG.3 (B)).

Then, enzyme activity of the POD combined to the well wall is measured to quantify collagen (FIG.3 (C)).

Also in this 1-step sandwich method, the following processes are required;
(1) Preparation of antigen
(2) Preparation of two types of monoclonal antibodies by using the aforesaid antigen
(3) Enzyme labelling of one of the monoclonal antibodies described above

10

(4) Coating of the another monoclonal antibody described above to a solid phase

(5) Actual measurement and (1) preparation of antigen and (2) preparation of monoclonal antibodies are carried out primarily like in the 3-step sandwich method described above.

For this reason, (3) Enzyme labelling of monoclonal antibody, (4) Coatation of monoclonal antibody to a solid phase, and (5) Actual measurement will be explained hereinafter.

### (3) Enzyme labelling of monoclonal antibody

#### (a) Method of preparing biotinylated antibody (Fab'-B)

A monoclonal antibody (JK 132; 10 mg/2 ml) solution prepared according to the 3-step sandwich method was dialyzed against 0.1 M acetate buffer solution (pH:4.2), a pepsin solution (produced by Berlinger Manheim Corp.; 20 mg/ml, 0.1 M acetate buffer solution: $50\mu$ l) was added to antibody solution above, and the mixture was left for 30 minutes under 37°C. Then the solution was centrifuged, the supernatant was applied to Sephacryl-S-200 column (Diameter 1.5 cm × 50 cm: eluate: 0.1 M phosphate buffer solution: pH 6.0), and F (ab')$_2$ fraction was obtained.

Dithiothreitol was added to this F (ab') fraction so that the concentration would be 20 mM, and the mixture was left for 1 hour at 37 °C for reaction. Then, the solution was centrifuged, the supernatant was applied to the Sephacryl-S-200 column (Diameter 1.5 cm × 50 cm, eluate : 0.1 M phosphate buffer (pH: 6.0), and F ab' fraction was obtained.

According to the method described in Anal. Biochem. 149, pp. 529 to 536 (1985), 3-(N-maleimidobuthylyl)-biocytin (MBB) was synthesized, the MBB solution (0.5 mg/ml 0.1 M phosphate buffer pH 6.0, 0.5 ml) was added to Fab' (1.66 mg/1.3 ml), and the mixture was left for 2 hours at the room temperature for reaction. The reaction solution was centrifuged, the supernatant was applied to the Sephadex-G-25 column (Diameter 1.5 cm × 30 cm, eluate: 0.1 M phosphate buffer pH:6.0), and biotinylated-Fab' was obtained.

#### (b) Method of preparing biotinylated antibody-avidin POD complex (Fab'-B-A-POD)

Avidin POD (2 mg; Produced by Vector Laboratories) was added to the Fab'-B (0.2 mg) prepared according to the method described in (a) above, and the mixture was left for 1 hour at 37°C for reaction. The reaction solution was centrifuged, the supernatant was applied to Sepharose-6B-column (Diameter 1.5 cm × 45 cm, eluate: 0.1 M HEPES buffer solution, pH: 7.0), and Fab'-B-A-POD was obtained.

### 4) Immobilization of monoclonal antibody to a solid phase

The monoclonal antibody (JK 199) obtained according to the method of preparing a monoclonal antibody (b) described above was diluted by 50 mM carbonate buffer solution (pH 9.6), and the concentration was adjusted to 15 $\mu$ g/ml. This antibody solution (150 $\mu$l) was put in wells on a plate (Produced by Nunk Corp.; Immunoplate I, 96 holes), and was left therein for one night. After the wells were washed with saline several times, 1% BSA(bovine serum albumin) solution (0.1 M phosphate buffer solution, pH 7.0: 400 $\mu$l) was added, the mixture was left for 2 hours at the room temperature, and IgG immobilized solid phase was obtained.

### 5) Actual measurement

Human type IV collagen (produced by Sigma Corp.) was dissolved in human serum not containing type IV collagen, and samples with the collagen concentration of 0 to 1000 ng/ml were prepared.

20 $\mu$l of this sample was put in a solid phase well (FIG.3 (A)), and furthermore 100 $\mu$l of Fab'-B-A-POD solution [ prepared by diluting the complex prepared in the process 3. 300 to 1,000 times: 50 mM HEPES buffer solution (pH 8.0: 1.0M potassium iodide, 4500U/ml heparin sodium, 0.2 % PA 100, 2.5 mM EDTA sodium contained] was added, the mixture was heated for 1.5 hours under 37°C, and after the reaction was complete, the reactant solution was washed away with a saline several times (FIG.3 (B)).

Then, a substrate solution (1 mg/ml ABTS, 0.01% hydrogen peroxide, 0.1 M citric acid buffer solution, pH 4.2) was added by 150 $\mu$ l, the mixture was heated for 10 minutes under 37°C, absorbance at 415 nm (main wavelength) and 650 nm (sub wavelength) was measured by microplate reader (FIG.3 (C)), and a calibration curve was prepared.

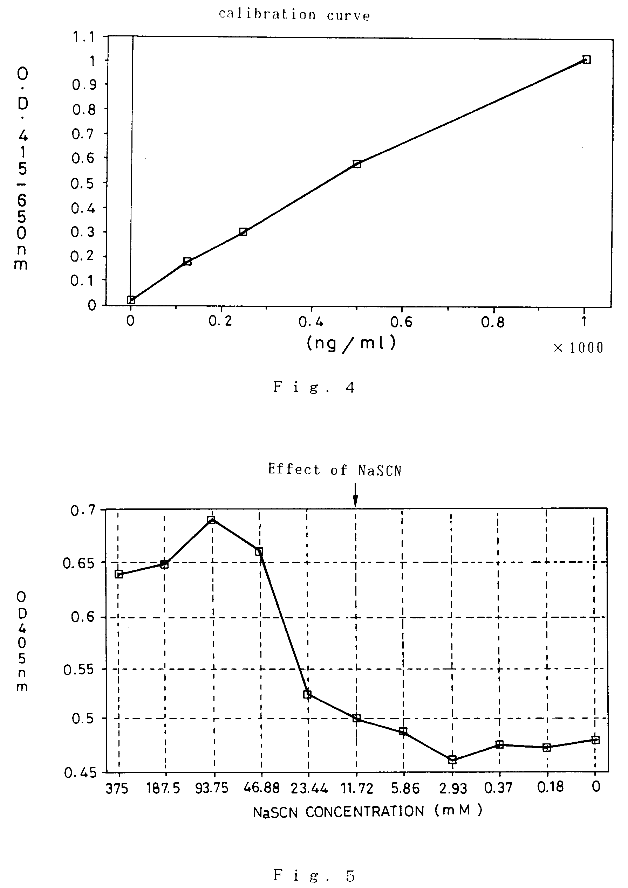

The result is shown in FIG.4. In case of collagen 1000 ng/ml, absorbance of approx. 1.0 was shown.

Influence provided by adding chaotropic ions

Influence provided by chaotropic ions according to the present invention will be explained hereinafter with reference to the EIA 1-step sandwich method and 3-step sandwich method.

(a) Investigation on 1-step sandwich method

Investigation for trichloride ion (trichloroacetic acid), iodine ion (potassium iodide), bromine ion (potassium bromide), nitric acid ion (sodium nitrate), chlorine ion (potassium chloride) and other substances similar to chaotropic ions (salicylic acid: sodium salicylate, P-nitrophenyl ion: P-nitrophenyl sodium phosphate) was carried out. The concentration of added substances were 0.1 M and 0.5 M respectively. It should be noted that, in case of trichloroacetic acid, the concentration were 0.5 and 1 % respectively. A sample to which any of these substances was not added was investigated as a control to check the effect of addition of these substances.

Like in the 1-step sandwich method described above, the aforesaid chaotropic ions were added during the immunoreaction.

As shown in Table 3, the effect of addition was observed in cases of iodine ion, perchloric acid ion, bromine ion, nitric acid ion, chlorine ion, and salicylic acid ion.

Then, investigation was carried out for the preferred concentration of sodium thiocyanate to be added , which is one of other substances recognized as effective when added, using purified type IV collagen as a sample to be assessed.

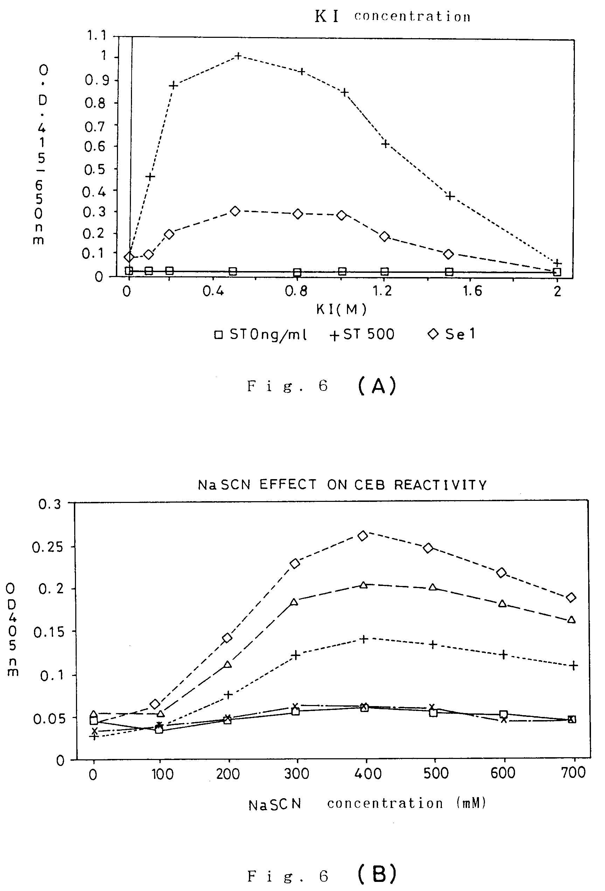

As a result, the effect of addition was observed when the concentration of the substance was 11.72 mM or more, as shown in FIG.5.

It should be noted that, also in case of salicylic acid ion, the effect of addition was observed when the concentration was in a range from 0.025 to 0.5 M.

Thus, when chaotropic ions are added, the concentration should preferably be 0.01 M or more.

Also, investigation was carried out for the concentration of iodine ion to be added, which was recognized as the most effective when added, using serum as a sample to be assesed.

As shown in FIG. 6 (A), an especially remarkable effect was observed when the concentration of the substance was in a range from 0.1 to 1.6 M.

Thus, when chaotropic ions are added during an immunoreaction, the concentration should preferably be in a range from 0.1 to 1.6 M.

Table 3  Effect of Addition of Chaotropic Ions

| Substance | When not added | Trichloroacetic acid | |
|---|---|---|---|
| Concentration | | 0.5 | 1% |
| ST    0 ng/ml | 0.025 | 0.024 | 0.025 |
| 1000 | 0.116 | 0.129 | 0.139 |
| CONT.serum | 0.1 | 0.151 | 0.154 |

| Substance | Na salicylate | | P-nitrophenyl phosphate Na | |
|---|---|---|---|---|
| Concentration | 0.1 M | 0.5 M | 0.1M | 0.5M |
| ST    0 ng/ml | 0.03 | 0.027 | 0.019 | 0.019 |
| 1000 | 0.891 | 0.119 | 0.074 | 0.019 |
| CONT.serum | 0.258 | 0.04 | 0.042 | 0.021 |

| Substance | KI | | KBr | |
|---|---|---|---|---|
| Concentration | 0.1 M | 0.5 M | 0.1M | 0.5M |
| ST    0 ng/ml | 0.017 | 0.020 | 0.021 | 0.022 |
| 1000 | 0.458 | 1.024 | 0.228 | 0.469 |
| CONT.serum | 0.095 | 0.284 | 0.146 | 0.424 |

| Substance | Na nitrate | | KCl | |
|---|---|---|---|---|
| Concentration | 0.1M | 0.5M | 0.1M | 0.5M |
| ST    0 ng/ml | 0.02 | 0.019 | 0.02 | 0.02 |
| 1000 | 0.158 | 0.368 | 0.134 | 0.317 |
| CONT.serum | 0.105 | 0.329 | 0.08 | 0.282 |

| Substance | $NaClO_4$ | |
|---|---|---|
| Concentration | 0.1M | 0.5M |
| ST    0 ng/ml | 0.017 | 0.025 |
| 1000 | 0.987 | 0.725 |
| CONT.serum | 0.205 | 0.330 |

(b) Investigation on 3-step sandwich method

Thiocyanic acid ions (sodium thiocyanate) were added to a sample to be assessed (serum) as chaotropic ions according to the 3-step sandwich method described above, and investigation was carried out using 5 types of serum, each having different collagen concentration.

As shown in FIG.6 (B), the measurement sensitivity of type IV collagen in serum rapidly raised when the concentration of sodium thiocyanate was more than around 0.2 M, and the sensitivity was maintained until the concentration raised up to 0.7 M. As the reactivity with an antibody drops when the concentration is too high, the concentration of the substance to be added should be in a range from 0.2 to 0.6 M, and preferably be in a range from 0.3 to 0.5 M.

Thus, when chaotropic ions are added for pretreatment a sample to be assessed, the concentration should preferably be in a range from 0.2 to 0.6 M.

As described above, when chaotropic ions were added, the reactivity of collagen with the antibody became remarkably high, and it became possible to carry out the reaction even at the temperature of around 37°C.

When considering the property of chaotropic ions to denature proteins, it can be considered that the effect provided by adding chaotropic ions according to this measuring method occurs because the immunoreaction becomes more active due to change of collagen's composition by the chaotropic ion which posesses protein denaturation effect. This suggests that the present invention is effective not only in measurement of collagen, but also in a quantifying method making use of an immunoreaction of substances in which the antigenicity becomes active due to partial denaturation of the antigen as well as in a method of quantifying substances in which sensitivity for chemical determination goes up via partial denaturation of the substances. And, as a result of it, it becomes possible by adding chaotropic ions that sample treating during an immunoreaction or when an immunoreaction is carried out after pretreatment the sample can be carried out under lower temperature.

Influence of addition of heparins

Influence of addition of heparins according to the present invention will be explained hereinafter over measurement of collagen with reference to the EIA 1-step sandwich method.

As heparins, heparin sodium, chondroitin salfate A sodium salt, chondroitin sulfate C sodium salt, potassium dextran sulfate, and pentosan polysulfate sodium salt were used. The measurement was carried out according to the procedure like in the 1-step sandwich method described above, and the heparins were added during the immunoreaction.

Results of addition of these substances are shown in Table 4. The effect of addition was observed in cases of heparin sodium, chondroitin sulfate A sodium salt, and chondroitin sulfate C sodium salt.

Table 4  Effect of Addition of Heparins

| Substance | When not added | Chondroitin sulfate A sodium salt | |
|---|---|---|---|
| Concentration | | 12.5 | 50 |
| ST     0 ng/ml | 0.021 | 0.022 | 1.057 |
|       1000 | 0.318 | 0.795 | 1.651 |
| CONT.serum | 0.047 | 0.191 | 0.655 |

| Substance | Chondroitin salfate C sodium salt | | Heparin Na |
|---|---|---|---|
| Concentration(mg/ml) | 12.5 | 50 | 26 |
| ST     0 ng/ml | 0.022 | 1.013 | 0.02 |
|       1000 | 0.939 | 1.468 | 0.838 |
| CONT.serum | 0.21 | 0.553 | 0.138 |

| Substance | Dextran sulfate K | | Pentosan Na polysulfate | |
|---|---|---|---|---|
| Concentration(mg/ml) | 12.5 | 50 | 12.5 | 50 |
| ST     0 ng/ml | 0.02 | 0.021 | 0.019 | 0.02 |
|       1000 | 0.369 | 0.333 | 0.357 | 0.432 |
| CONT.serum | 0.035 | 0.038 | 0.041 | 0.047 |

Then investigation was carried out for preferable densities of heparin sodium, chondroitin sulfate A sodium salt, and chondroitin sulfate C sodium salt, all of which had been recognized as effective when added.

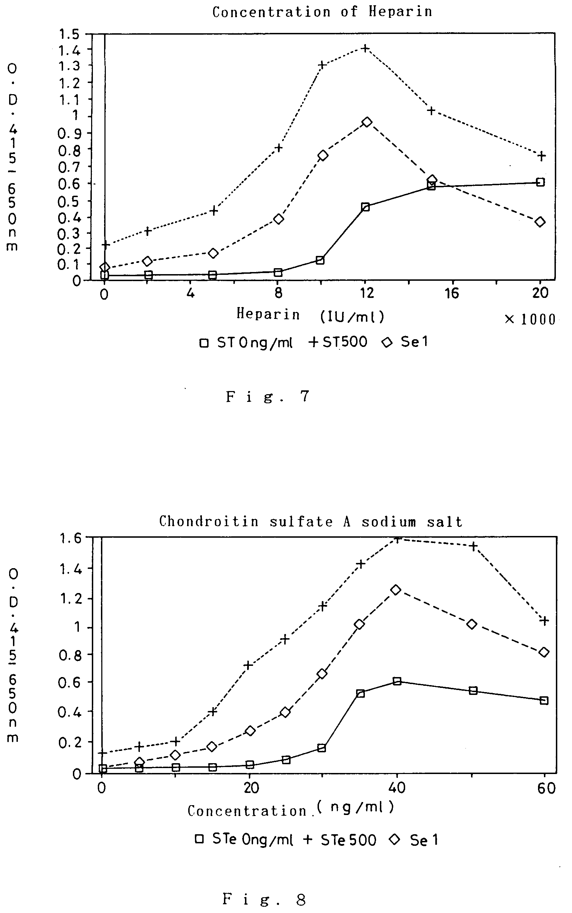

Results of the investigation on heparin sodium, chondroitin sulfate A sodium salt, and chondroitin sulfate C sodium salt are shown in FIG.7, FIG.8 and FI.9 respectively.

As clearly shown in these figures, the effect of addition of heparin sodium was observed when the concentration was in a range from 250 to 15000 U/ml, while those of chondroitin sulfate A sodium salt and chondroitin sulfate C sodium salt were observed when the concentration was in a range from 5 to 60 mg/ml.

These effects are shown more clearly in the existence of chaotropic ions.

Influence of addition of chelating agent

Then, investigation was carried out for the effect of a chelating agent to prevent the activity for suppressing sandwich ELISA reaction in a sample from appearing.

Namely, investigation was carried out for prevention of appearance of suppressing sandwich ELISA reaction in a sample, using EDTA and EGTA as chelating agents.

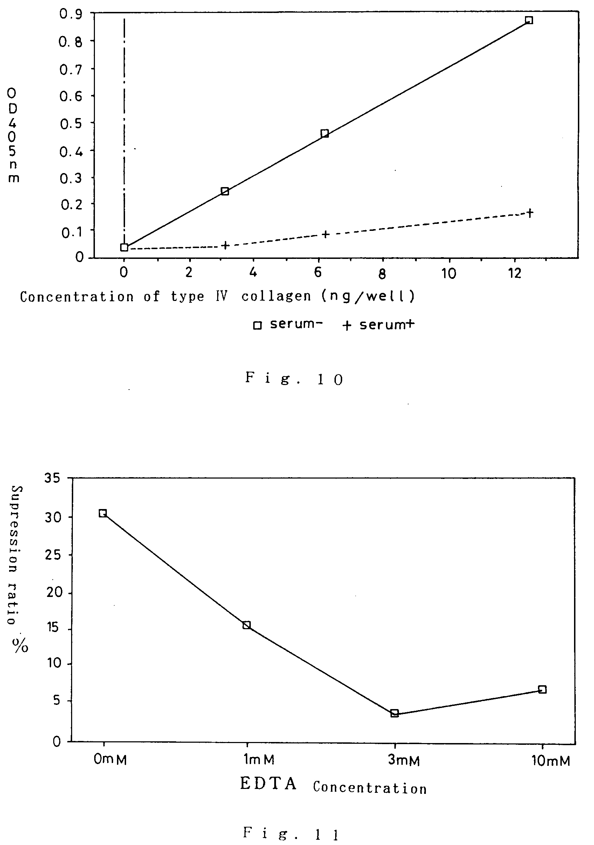

FIG.10 shows an analytical curve (□-□) of human type IV collagen in the absence of serum and also an analytical curve ( + • • • + ) of human type IV collagen under the existence of serum stored in a frozen state.

As clearly shown in this figure, when 25$\mu$l of serum obtained from a normal man was added to 100 $\mu$l of the sample, it was observed that appearance of the activity was suppressed by 80 %.

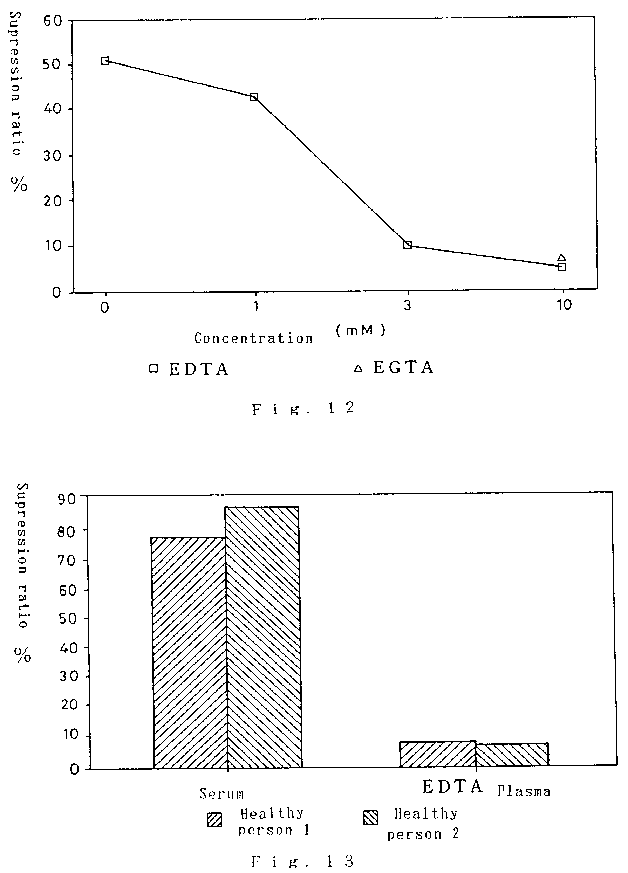

In contrast, when EDTA was added immediately after separation of serums and an experiment on the effect to suppress appearance of the activity without freezing the sample (□-□ in FIG.11), and when the same experiment was carried out after the sample was stored in a frozen state (□-□ in FIG.12), a degree of suppression by serums remarkably dropped as the EDTA concentration increased from 3 mM to 10 mM.

The same tendency was observed also in case of EGTA (Δ in FIG.12).

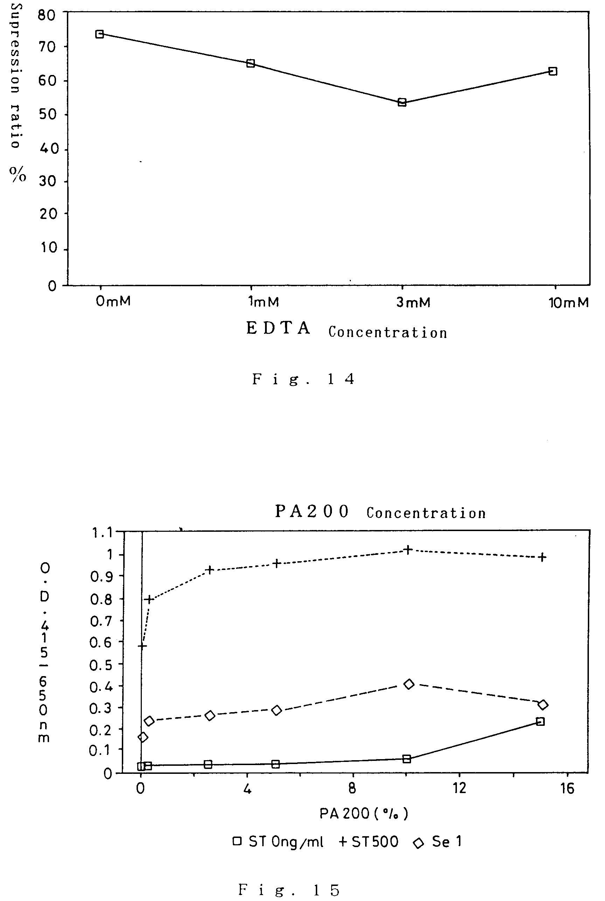

Also, appearance of the activity for suppression was remarkably obstructed in plasma prepared by obstructing blood coagulation when the blood was taken out (FIG.13).

On the other hand, when EDTA was added to a blocking buffer solution, the effect was weak, and a suppression degree by serums dropped when the concentration of EDTA was 3 mM or more (FIG.14).

Influence of addition of gelatins

Next, influence of addition of gelatins, which is specific to the present invention, will be explatined hereinafter over measurement of collagen, with reference to the EIA 1-step sandwich method and 3-step sandwich method.

(a) Investigation on 1-step sandwich method

Investigation was carried out for PA10, 100, 200 and PE 20, 50 as gelatins. The concentrations of added gelatin were 1% and 4 %. The measurement was carried out according to the same procedure as that in the 1-step sandwich method described above, and the gelatins were added during the immunoreaction.

As shown in Table 5, the excellent effect was observed in case of PA 200.

### Table 5  Effect of Gelatins

| Substance | PA 10 | | PE 20 | |
|---|---|---|---|---|
| Concentration (%) | 1 | 4 | 1 | 4 |
| ST     0 ng/ml | 0.012 | 0.012 | 0.021 | 0.015 |
|         1000 | 0.698 | 0.39 | 0.744 | 0.66 |
| CONT.serum | 0.13 | 0.06 | 0.258 | 0.169 |

| Substance | PE 50 | | PA 100 | |
|---|---|---|---|---|
| Concentration (%) | 1 | 4 | 1 | 4 |
| ST     0 ng/ml | 0.016 | 0.014 | 0.016 | 0.017 |
|         1000 | 0.824 | 0.665 | 0.831 | 0.792 |
| CONT.serum | 0.169 | 0.152 | 0.157 | 0.171 |

| Substance | PA 200 | |
|---|---|---|
| Concentration (%) | 1 | 4 |
| ST     0 ng/ml | 0.025 | 0.03 |
|         1000 | 1.066 | 1.104 |
| CONT.serum | 0.196 | 0.304 |

Then, investigation was carried out for a relation between a concentration of added PA 200, which was recognized as extremely effective when added, and the effect of its addition.

The result is shown in FIG.15. As clearly shown in this figure, it was observed that addition of PA 200 was effective when the concentration of added PA 200 was in a range from 0.2 to 15 %. This suggests that addition of gelatins improves the immunological activity.

(b) Investigation on the 3-step sandwich method and a method in which a sample is pretreated

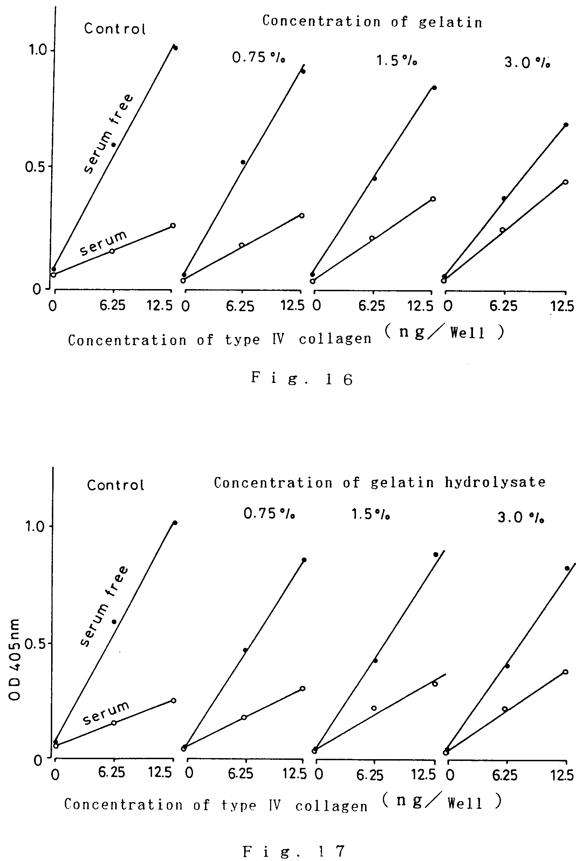

When gelatin (produced by Sigma Corp., type A) was added to a blocking solution, as shown in FIG. 16, the degree of suppression by the serum drops as the concentration of gelatin increases from 0.75 to 1.5 and then 3.0 %.

The same tendency is also observed in case of hydrolysates of gelatins (FIG.17).

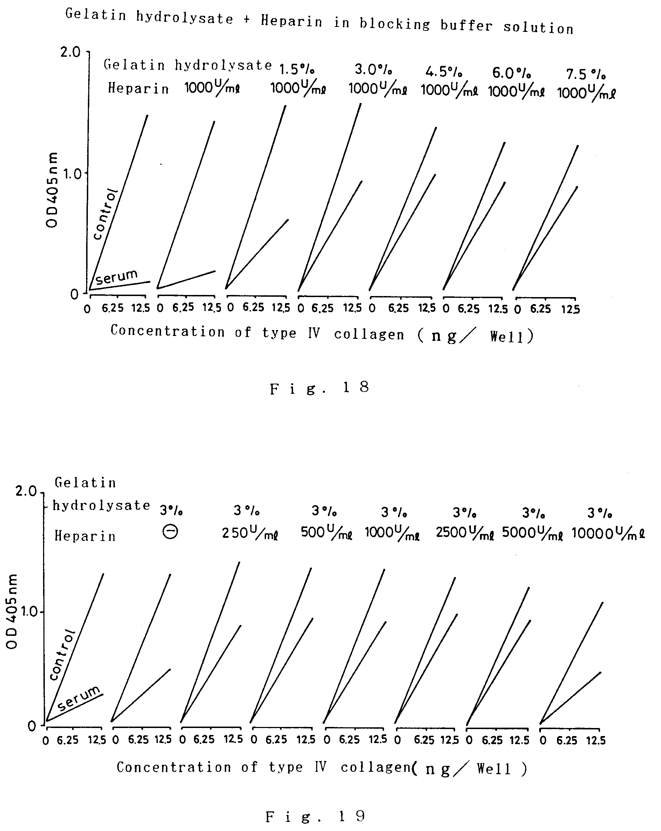

When gelatin (hydrolysate of a gelatin) and heparin sodium coexisted in a blocking buffer solution, a more excellent effect for obstructing suppression was observed (FIG.18).

As clearly shown in the figure, when the concentration of heparin was fixed at 1000 U/ml and the concentration of gelatin hydrolysate was increased from 1.5 to 3.0,4.5,6.0 and finally up to 7.5 %, the degree of suppression by serum dropped almost in proportion to the concentration until the concentration raised up to 4.5%. For this reason, the concentration of gelatins should preferably be in a range from 1.5 to 4.5 %.

FIG.19 shows a case where the concentration of gelatin hydrolysate was coated at 3 % and the concentration of heparin was raised from 250 to 500, 1000, 2500, and 5000 U/ml, and it can be understood that a degree of suppression by serum drops when the concentration of heparin is in a range from 250 to 5000 U/ml. For this reason, practically the concentration of heparin should preferably be in a range from 250 to 5000 U/ml.

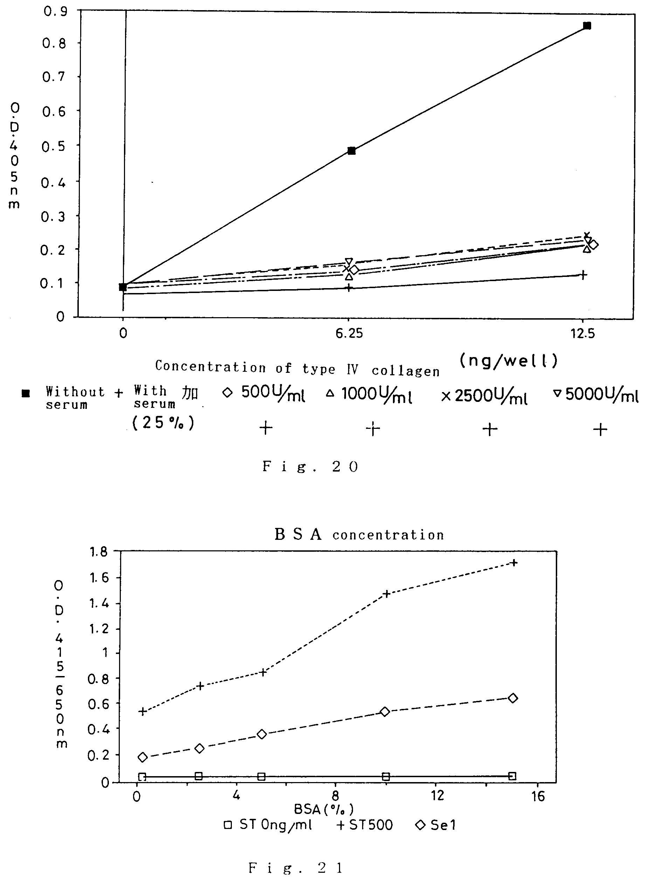

FIG.20 shows the effect of adding only heparin sodium, and the solid line ■ shows a result of the experiment when serum were not added, the solid line + shows a result of the experiment when serum were added, and the dotted lines ◊, △, ×, and ∇ show results of the experiments when heparin sodium was added by 500, 1000, 2500, and 5000 U/ml respectively.

As clearly shown in the figure, in the 3-step sandwich method, or when pretreatment is carried out, a suppression by serum can hardly be obstructed by adding only heparin.

This result suggests that heparins have both an effect to promote the immunological activity as described above and an effect to improve the effect provided by adding gelatins.

Influence of addition of albumin

Then, investigation for influence of addition of albumin was carried out, using the 1-step sandwich method.

As albumin, bovine serum albumin (BSA) was used. The concentration of added BSA was 0.2% or 4%. The result is shown in Table 6.

Table 6

| Effect of Albumin | | |
|---|---|---|
| Substance | BSA | |
| Concentration(%) | 0.2 | 4 |
| ST 0 mg/ml | 0.02 | 0.023 |
| 1000 | 0.784 | 0.928 |
| CONT.serum | 0.264 | 0.309 |

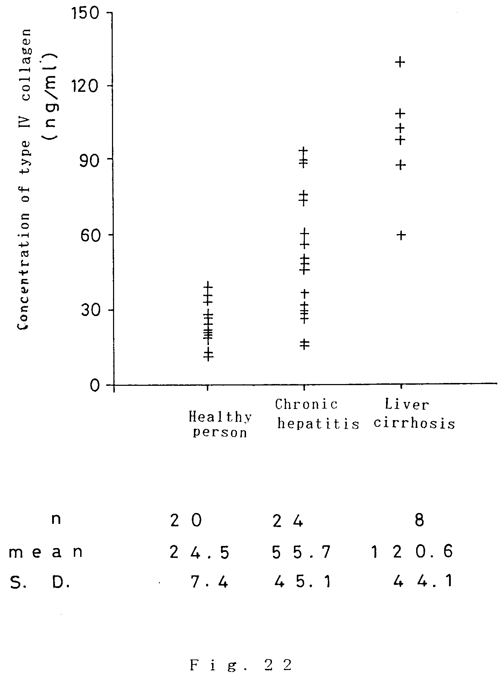

Based on the results as shown in Table 6, it was recognized that measurement sensitivity raised due to addition of albumin. So investigation for concentration of added albumin was carried out. The result is shown in Fig. 21. It was recognized that the effect became higher as the concentration of albumin went up.

Embodiment 1 Quantification of type IV collagen in serum

According to the same procedure as that in the 3-step sandwich method described above, 25 $\mu$l of a standard sample, or 25$\mu$l of serum taken from a patient as a sample to be assessed was put in each well coated antibody JK199 together with 75 $\mu$l of a saline (pH 7.4, GHE buffer solution ) containing 4.5% gelatin hydrolysate, 1000 U/ml of heparin sodium, 6.66 mM of EDTA and 533 mM of NaSCN respectively, and the sandwich ELISA reaction was carried out at 37°C.

A recovery ratio of almost 100 % was obtained in the recovery experiments using serum from patients and healthy persons. Also the results of intra- and interassay showed good reproducibility.

A quantity of type IV collagen in serum was checked in 20 cases of healthy persons, 24 cases of chronic hepatitis and 8 cases of liver cirrhosis, and distribution by disease were checked.

The result is shown in FIG.22.

The average was 24.5 ng/ml in cases of healthy persons, 55.7 ng/ml in case of chronic hepatitis, and 120.6 ng/ml in case of liver cirrhosis, and the value of type IV collagen raised as liver fibrosis proceeded.

As shown in FIG.22, the values obtained through measurement according to the present invention on the concentration of human collagen in serum obtained from patients suffering from liver diseases are clearly higher than those on serum obtained from healthy people. For this reason, it is possible to detect liver diseases, especially liver fibrosis, liver cancer and digestive organ cancer metastasized to liver in their early stage without carrying out biopsy which is troublesome to the patients, by measuring the concentration of human type IV collagen in human blood according to the method by the present invention.

Embodiment 2 Quantification of type IV collagen in serum (1-step sandwich method)

Intra-assay and recovery test were carried out according to the same procedure as that in the 1-step sandwich method described above for basic assessment of the 1-step sandwich method as a measuring method (n = 7). In the recovery test, 2 types of serum were added by the same amount, and a recovery rate was obtained from the values on each serum and the value on the mixed serum (n = 6).

The result of the experiment on intra-assay is shown in Table 7. The coefficient of variation (C.V) was approximately 10% in case of the serums and approximately 5 % in the standard serum, so that the reproducibility is high.

Table 7

| Intra-assay variation (1MKI 4500 U/ml heparin 90 - 10 min) | | | | | |
|---|---|---|---|---|---|
| Sample | ST 125 | ST 250 | Se 1 | Se 2 | Se 3 |
| 1 | 0.249 | 0.443 | 78 | 97 | 305 |
| 2 | 0.255 | 0.412 | 101 | 113 | 361 |
| 3 | 0.243 | 0.445 | 96 | 109 | 350 |
| 4 | 0.255 | 0.44 | 97 | 88 | 296 |
| 5 | 0.274 | 0.467 | 101 | 83 | 297 |
| 6 | 0.236 | 0.455 | 76 | 112 | 271 |
| 7 | 0.237 | 0.461 | 92 | 90 | 335 |
| X | 0.249 | 0.446 | 91.57 | 98.85 | 316.4 |
| S.D | 0.014 | 0.019 | 9.237 | 13.39 | 34.98 |
| CV (%) | 5.271 | 4.039 | 11.39 | 12.57 | 10.35 |

Results of the recovery test are shown in Table 8. The recovery ratio was in a range from 91.7 to 110.1 % (Average = 101.5 %), which is very high.

Table 8

| Recovery When Different Types of Serum were Mixed 1MKI 4500 U/ml Heparin | | | | |
|---|---|---|---|---|
| Measured values for | | | Theoretical value | Recovery ratio(%) |
| SeA | Seb | A + B | | |
| 35 | 175 | 106 | 105 | 100.5 |
| 27 | 163 | 102 | 95 | 107.4 |
| 15 | 145 | 75 | 80 | 93.8 |
| 33 | 203 | 131 | 119 | 110.1 |
| 26 | 191 | 100 | 109 | 91.7 |
| 32 | 230 | 138 | 131 | 105.3 |

Also, investigation for distribution by disease was carried out, and the result similar to that in embodiment 1 was obtained.

Depending on the facts described above, it can be said that this method is excellent as a measuring method.

Embodiment 3 Tool kit for measurement of collagen in serum

Based on the results described abobve, a tool kit for measurement of collagen in serum, in which the 1-step sandwich method was applied, was manufactured.

(a) Antibody coated solid phase

Antibody JK 199 (IgG) coating plate

The method of coating antibodies is the same as that in the aforesaid 1-step sandwich method.

(b) Agent for treating samples

The Fab'-B-A-POD is diluted by using a sample treating agent, and the product is used as an agent for treating samples. 50 mM-HEPES, 1.0M - KI, 2.5 mM-EDTA, 0.2 % PA-100, 4500 IU/ml-Heparin sodium, and 0.25 mg/ml-mouse IgG, all with pH of 8.0, are available as a solution for treating samples.

(c) Enzyme substrate solution

The substrate 1 mg/ml-ABTS and $35\mu$l/dl-$H_2O_2$ are dissolved by a substrate dissolving solution, and the product is used as an enzyme substrate solution. The substrate dissolving solution is a mixture of 0.1 M citric acid and 1mM-EDTA and the pH is 4.2.

20 $\mu$l of a sample to be assessed and 100 $\mu$l of the agent for treating samples were added to the aforesaid antibody coated solid phase and left therein for 1.5 hours under 37°C.

Then, the solid phase was washed 6 times with saline, and 150 $\mu$l of the enzyme substrate solution was added and left therein for 10 minutes under 37°C.

And, the resultant solution was measured by colorimetric determination at 415 nm and 650 nm.

Results of measurement with this kit fairly coincide with the actual collagen concentration, and also life of the kit was long.

Embodiment 4 Tool kit for measurement of collagen in serum

A tool kit for measurement of collagen in serum, in which the 1-step sandwich method was applied, was manufactured.

(a) Antibody coated solid phase

Antibody JK 199 (IgG) coating plate

The method for coating antibodies is the same as that in the aforesaid 1-step sandwich method.

(b) Agent for treating samples

Fab'-B-A-POD solution was diluted by using a sample treating solution, and the product was used as an agent for treating samples. As a sample treating solution, D-PBS', 1.0 M-KI, 2.5 mM-EDTA, 0.2 % PA-100, 4500 IU/ml-heparin sodium, and 0.25 mg/ml-mouse IgG, all with pH of 7.0, are available.

(c) Enzyme substrate solution

The substrate 1 mg/ml-ABTS and $35\mu l/dl$-$H_2O_2$ are dissolved by a substrate dissolving solution, and the product is used as an enzyme substrate solution. The substrate dissolving solution is a mixture of 0.1 M citric acid and 1mM-EDTA and the pH is 4.2.

20 $\mu l$ of a sample to be assessed and 100 $\mu l$ of the agent for treating samples were added to the aforesaid antibody coated solid phase and left therein for 1.5 hours at 37°C.

Then, the solid phase was washed 6 times with saline, and 150 $\mu l$ of the enzyme substrate agent was added and left therein for 10 minutes at 37°C.

And, the resultant solution was measured by colorimetric determination at 415 nm and 650 nm.

Embodiment 5 Tool kit for measurement of collagen in serum

A tool kit for measurement of collagen in serum, in which the 1-step sandwich method was applied, was manufactured.

(a) Antibody coated solid phase

Antibody JK 199 (IgG) coating plate

The method for coating antibodies is the same as that in the aforesaid 1-step sandwich method.

(b) Agent for treating samples

Fab'-B-A-POD is diluted by a sample treating solution, and the product is used as an agent for treating samples. As a sample treating solution, 50 mM-HEPES, 0.1M sodium salicylate, 2.5 mM-EDTA, 0.2% PA-100, 4500 IU/ml-heparin sodium, and 0.25 mg/ml-mouse IgG, all with pH 8.0, are available.

(c) Enzyme substrate solution

The substrate 1 mg/ml ABTS and 35 $\mu l/dl$-$H_2O_2$ are dissolved by a substrate dissolving solution, and the product is used as an enzyme substrate solution. The substrate dissolving solution is a mixture of 0.1 M citric acid and 1mM-EDTA and the pH is 4.2.

20 $\mu l$ of a sample to be assessed and 100 $\mu l$ of the agent for treating samples were added to the aforesaid antibody coated solid phase and left therein for 1.5 hours at 37°C.

Then, the solid phase was washed 6 times with saline, and 150 $\mu l$ of the enzyme substrate agent was added and left therein for 10 minutes at 37°C.

And, the resultant solution was measured by colorimetric determination at 415 nm and 650 nm.

Embodiment 6 Tool kit for measurement of collagen in serum

A tool kit for measurement of collagen in serum, in which the 1-step sandwich method was applied, was manufactured.

(a) Antibody coated solid phase with agent for treating samples frozen and dried therein

A solid phase to which the antibody JK 199 (IgG) was prepared according to the same procedure as that in the aforesaid 1-step sandwich method. Then, an agent for treating samples was added to the antibody coated solid phase, and freeze-drying was carried out.

The freeze-drying was carried out according to an ordinary method.

When adjusting the agent for treating samples, Fab'-B-A-POD is diluted by using a sample treating solution. As the solution for treating samples, 50 mM-HEPES, 1.0 M-KI, 2.5 mM-EDTA, 0.2 % PA-100, 4500 IU/ml-heparin sodium, and 0.25 mg/ml-mouse IgG, all with pH 8.0, are available.

(b) Enzyme substrate solution

The substrate 1 mg/ml ABTS and 35 $\mu$l/dl-$H_2O_2$ are dissolved by a substrate dissolving solution, and the product is used as an enzyme substrate solution. The substrate dissolving solution is a mixture of 0.1 M citric acid and 1mM-EDTA and the pH is 4.2.

With the tool kit above, 20 $\mu$l of a sample and 100 $\mu$l of purified water were added to the aforesaid antibody coated solid phase with agent for treating samples frozen and dried therein, and left therein for 1.5 hours at 37°C.

Then, the solid phase was washed 6 times with saline, and then 150 $\mu$l of the enzyme substrate solution was added and left therein for 10 minutes at 37°C.

And, the reactant solution was measured by colorimetric determination at the wavelengths of 415 nm and 650 nm.

It is well known that detection of liver fibrosis, liver cancer and digestive organ cancer metastasized to liver is difficult in measurement for ZTT (zinc sulfate turbid reaction), GOT (glutamine oxaloacetic transaminase), GPT (glutamine pyruvic acid transaminase), ALP (alkaline phosphatase), LDH (lactate dehydrogenase ), and $\gamma$-GTP (glutamine transpeptitase), all of which are often used in determination of liver functions, and it can be expected that these types of disease can also be detected in their early stage by measuring human type IV collagen in blood according to the measuring method according the present invention.

As it is possible to diagnose liver fibrosis, liver cancer, and digestive organ cancer metastasized to liver by measuring the concentration of human type IV collagen according to the measuring method according to the present invention, the present invention has an extremely high utility.

As described above, it is possible to largely improve the reactivity between an antibody and collagen by adding chaotropic ions according to the present invention, so that measurement sensitivity of collagen is largely raised. Also by treating a sample to be assessed with a chelating agent for bivalent cations, it is possible to suppress appearance of inhibitory activity or obstruction by other substances in measurement of collagen.

**Claims**

1. A method of measuring collagen by an immunological measuring method using monoclonal antibodies against collagen, characterized in that a sample to be assessed is treated with chaotropic ions.

2. The collagen measuring method according to claim 1, characterized in that chaotropic ions are added so that the concentration will be 0.01 M or more.

3. The collagen measuring method according to claim 1 or claim 2, characterized in that a sample to be assessed is treated with heparins.

4. The collagen measuring method according to claims 1 to 3 , characterized in that a sample to be assessed is treated with a chelating agent.

5. The collagen measuring method according to claims 1 to 4, characterized in that a sample to be assessed is treated with gelatins.

6. The collagen measuring method according to claims 1 to 5, characterized in that a sample to be assessed is treated with albumins.

7. The collagen measuring method according to claims 1 to 6, characterized in that chaotropic ions are added during an immunoreaction of a serum sample to be assessed so that the concentration of added chaotropic ions will be in a range from 0.1 to 1.6 M.

8. The collagen measuring method according to claims 1 to 6, characterized in that chaotropic ions are added when a sample to be assessed is pretreated so that the concentration of added chaotropic ions will be in a range from 0.2 to 0.6 M.

9. The collagen measuring method according to claim 3, characterized in that heparins are added during an immunoreaction of a sample to be assessed so that the concentration of added heparins will be in a range from 250 to 15000 U/ml and/or from 5 to 60 mg/ml.

10. The collagen measuring method according to claim 3, characterized in that heparins are added when a sample to be assessed is pretreated so that the concentration will be in a range from 250 to 5000 U/ml.

11. The collagen measuring method according to claim 4, characterized in that a sample to be assessed is treated with 1 to 10 mM of a chelating agent for bivalent cations.

12. An collagen measuring method by an immunological measurement using monoclonal antibodies against collagen, characterized in that a sample to be assessed is treated with gelatins.

13. The collagen measuring method according to claim 12, characterized in that heparins are also used together with gelatins for treating a sample to be assessed.

14. A tool kit for measuring collagen including;
   an antibody coated solid phase to which an monoclonal antibody against collagen is coated,
   an agent for treating samples which contains an enzyme-labelled monoclonal antibody against collagenand chaotropic ions, reacts to a sample to be assessed as well as to the aforesaid antibody coated solid phase, and
   an enzyme substrate solution which contains a substrate for the enzyme of the aforesaid enzyme-labelled monoclonal antibody.

15. A tool kit for measuring collagen, including;
   a reaction phase where an enzyme-labelled monoclonal antibody against collagen, chaotropic ions, and a carrier to which an monoclonal antibody against collagen has been coated, coexist, and
   an enzyme substrate solution containing a substrate for the enzyme of the aforesaid-enzyme labelled monoclonal antibody.

(A)

(B)

(C)

(D)

JK199

Biotinylated JK132

Avidin-POD

Fig. 1

F i g . 2

(A)

(B)

(C)

JK199

Antigen

Fab'- B - A - POD ( J K 132 )

Fig. 3

calibration curve

F i g . 4

Effect of NaSCN

F i g . 5

26

Fig. 6 (A)

Fig. 6 (B)

**Concentration of Heparin**

□ ST 0ng/ml + ST 500 ◇ Se 1

F i g . 7

**Chondroitin sulfate A sodium salt**

□ STe 0ng/ml + STe 500 ◇ Se 1

F i g . 8

28

Fig. 9

Fig. 10

Fig. 11

F i g . 1 2

F i g . 1 3

Fig. 14

Fig. 15

Fig. 16

Fig. 17

Gelatin hydrolysate + Heparin in blocking buffer solution

F i g . 1 8

F i g . 1 9

Concentration of type IV collagen (ng/well)

■ Without serum   + With serum 加 (25%)   ◇ 500U/ml   △ 1000U/ml   × 2500U/ml   ▽ 5000U/ml
                      +            +            +            +

F i g . 2 0

B S A concentration

BSA (%)

□ ST 0ng/ml   + ST500   ◇ Se1

F i g . 2 1

35

Fig. 2 2

# INTERNATIONAL SEARCH REPORT

International Application No PCT/JP92/00328

**I. CLASSIFICATION OF SUBJECT MATTER** (if several classification symbols apply, indicate all) ⁶

According to International Patent Classification (IPC) or to both National Classification and IPC

Int. $Cl^5$   G01N33/577, G01N33/53, G01N33/531

**II. FIELDS SEARCHED**

Minimum Documentation Searched ⁷

| Classification System | Classification Symbols |
|---|---|
| IPC | G01N33/577, G01N33/53, G01N33/531 |

Documentation Searched other than Minimum Documentation
to the Extent that such Documents are Included in the Fields Searched ⁸

| | |
|---|---|
| Jitsuyo Shinan Koho | 1926 – 1991 |
| Kokai Jitsuyo Shinan Koho | 1971 – 1991 |

**III. DOCUMENTS CONSIDERED TO BE RELEVANT** ⁹

| Category * | Citation of Document, ¹¹ with indication, where appropriate, of the relevant passages ¹² | Relevant to Claim No. ¹³ |
|---|---|---|
| A | JP, A, 2-1553 (Fuji Chemical Industries, Ltd.), January 5, 1990 (05. 01. 90) | 1-15 |
| A | JP, A, 63-78067 (Shiseido Co., Ltd.), April 8, 1988 (08. 04. 88), (Family: none) | 1-15 |
| A | JP, A, 63-246396 (Shiseido Co., Ltd.), October 13, 1988 (13. 10. 88), (Family: none) | 1-15 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| May 18, 1992 (18. 05. 92) | June 9, 1992 (09. 06. 92) |

| International Searching Authority | Signature of Authorized Officer |
|---|---|
| Japanese Patent Office | |

Form PCT/ISA/210 (second sheet) (January 1985)